# EUROPEAN PATENT SPECIFICATION

(11) **EP 1 406 574 B1**
(45) Date of publication and mention of the grant of the patent: **26.09.2007**
(21) Application number: 02748846.9
(22) Date of filing: 04.07.2002
(51) Int. Cl.: A61K 8/49, A61Q 5/00

(54) **SKIN TREATMENT**
HAUTBEHANDLUNGSMITTEL
TRAITEMENT DE LA PEAU

(30) Priority: 18.07.2001 EP 01306165
(43) Date of publication of application: 14.04.2004
(73) Proprietor: UNILEVER PLC, London EC4P 4BQ (GB); UNILEVER N.V., 3013 AL Rotterdam (NL)
(72) Inventor: BAILEY, Peter L., Unilever Research Port Sunlight, Wirral, Merseyside CH63 3JW (GB); HARDING, Clive R. Unilever R&D Edgewater, New Jersey 07020 (US); MOORE, Alison, Unilever R & D Colworth, Bedford, Bedfordshire MK44 1LQ (GB); ROGERS, Julia S., Unilever R & D Colworth, Bedford, Bedfordshire MK44 1LQ (GB)
(74) Representative: Tansley, Sally Elizabeth
(86) International application number: PCT/EP2002/007529
(87) International publication number: WO 2003/007900

(56) References cited:
- EP-A- 0 925 779
- WO-A-96/29983
- WO-A-02/067880
- DE-A- 19 537 509
- US-A- 5 972 920
- US-A- 6 071 543
- DATABASE CAPLUS [Online] CHEMICAL ABSTRACTS SERVICE, COLUMBUS, OHIO, US; retrieved from STN Database accession no. 1982:129566 XP002188678
- DATABASE EMBASE [Online] ELSEVIER SCIENCE PUBLISHERS, AMSTERDAM, NL; retrieved from CAPLUS Database accession no. 92124811 XP002188679

## Description

This invention relates to treatment of the human scalp.

It is well-known that metal pyrithiones, such as zinc pyrithione, can be used in the treatment of dandruff. The conventional theory behind the activity of zinc pyrithione (ZnPTO) in treating dandruff is that the compound acts as an antimicrobial agent, killing the microbes that are responsible for causing the dandruff.

The present invention is based on the surprising finding that metal pyrithiones can act to increase the level of lipids in the stratum corneum of the scalp. It was not previously recognised that metal pyrithiones had activity other than as antimicrobial agents when applied to the scalp or to the skin in general.

Accordingly, the present invention provides the use of a metal pyrithione in the manufacture of a composition for increasing the level of lipids in the skin of the scalp.

The lipids of the scalp, the levels of which may be increased in the present invention, include triglyceride compounds, cholesterol, ceramides and mixtures thereof. The lipids are naturally present in the stratum corneum of the skin and the action of the metal pyrithione in the present invention serves to increase the overall total amount of these naturally occurring compounds that is present in the stratum corneum of the scalp.

Preferably, the lipids are the free, intercellular lipids that occur naturally in the stratum corneum of the skin.

The present invention may be selective for certain lipids. For example, it has surprisingly been found that, in the present invention, it is possible to decrease the total amount of ceramides 3 and 6ii relative to the amount of ceramide 3a as a proportion of the total ceramide content.

The present invention may involve an increase in the level of lipids on the skin, of the human scalp. Either all or part of the scalp may be treated in the present invention.

The present invention preferably involves application of the metal pyrithione to the scalp of a person suffering from dandruff. However, the present invention does not relate exclusively to increasing the level of lipids concomitantly with the treatment of dandruff and also contemplates increasing the level of lipids in the stratum corneum of the scalp of people not suffering from dandruff at the time that the metal pyrithione is applied to the scalp.

By increasing the level of lipids in the stratum corneum of the skin, the present invention has the effect of strengthening the skin. By the term strengthen the skin, and related terms used herein, we mean that the resistance of the skin to penetration by water is increased ie, the water permeability barrier of the skin is strengthened. By strengthening the water permeability barrier of the skin, the resistance of the skin to penetration by irritants, such as *Malassezia,* is increased. The strengthening of the skin may include an increase in the physical strength of the body of the skin (such as increased elasticity) and/or an increase in the ability of the surface of the skin to act as a barrier in repelling irritants (eg, by becoming more hydrophobic). The strengthening of the skin may comprise enhanced epithelial differentiation in the skin and/or an increased quality of the stratum corneum.

Increasing the level of lipids in the stratum corneum of skin, according to the present invention, can therefore have a number of beneficial effects for the skin.

The method of the invention preferably comprises the steps of:
(a) contacting the skin with water;
(b) applying to the skin a metal pyrithione;
(c) rinsing excess metal pyrithione from the skin; and
(d) determining the extent to which the level of lipids in the skin has increased.

Steps (a), (b) (c) are typically carried out in an analogous manner to the conventional treatment of hair by shampooing and/or conditioning. In step (c), the excess metal pyrithione that is rinsed from the skin is the metal pyrithione that has not deposited from the composition onto the skin. Step (d) may involve a determination by the user of the product by visual observation and/or by sensing the degree to which the level of lipids in the skin has increased, for example by sensing the elasticity of the skin. Alternatively, step (d) may involve a physical measurement of the level of lipids in the skin (eg, by removing flakes of skin onto an adhesive substrate such as a strip of adhesive tape, extracting free lipids from the flakes using a solvent for the lipids and analysing the amount of lipids eg, as the amount of lipids per weight of protein present) and/or of the extent to which the scalp has been strengthened, for example as measured either by corneosurfametry (CSM), transepidermal water loss (TEWL) or corneometry.

The insoluble metal pyrithione may be represented by the following general formula: 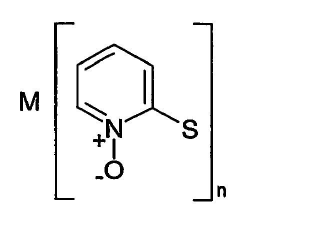 in which M is a polyvalent metal ion and n corresponds to the valency of M.

Preferred examples of M include magnesium, barium, strontium, zinc, cadmium, tin and zirconium. Especially preferred is zinc.

The metal pyrithione may have any particle form suitable for use in a composition for topical application to the skin. For example, the metal pyrithione may be in the form of amorphous or crystalline particles having a range of different particle sizes.

The metal pyrithione may, for example, be in the form of particles having a size distribution in which at least about 90% of the particles have a size of up to 100 microns, more preferably up to 50 microns, even more preferably up to 10 microns, most preferably 5 microns or less, for example the size distribution may be such that at least about 90% of the particles have a size of 1 micron or less. It is thought that smaller sizes enable the antimicrobial particles to be delivered down to the hair follicle, leading to a better efficacy.

Various methods for producing fine particles of metal pyrithione are described, for example, in EP-A-0 173 259. Suitable methods for determining particle size are described in that document.

The insoluble metal pyrithione may be made up of one particulate form or two or more different particulate forms.

Other suitable particulate forms for the metal pyrithione include platelets and needle-shaped particles. Platelets of zinc pyrithione are described in EP-A-0034385. The needle-shaped particles are preferably of the type described in WO99/66886. For needle-shaped particles preferably at least 50% by number of the particles are needle-shaped particles having a length of between 1µm and 50µm.

The metal pyrithione may be applied to the skin in the form of any composition that is suitable for topical application to the skin. Such compositions include those primarily intended for application to the hair, but which also have the effect of being applied to the scalp to some extent, such as hair treatment compositions including shampoos and conditioners.

The amount of metal pyrithione incorporated into the compositions may depend on the type of composition and the exact nature of the material used. A preferred amount of pyrithione is from about 0.001% to about 5% by weight of the total composition, more preferably from about 0.05% to about 3% by weight, most preferably between 0.1% and 1% by weight.

The compositions that may be used in the invention are preferably aqueous based. The composition suitably comprise water in amount of from about 20% to about 99%, more preferably from about 30% to about 80%, by weight of the total composition.

The compositions that are used in the invention are rinse-off compositions, i.e., suitable for applying to the hair and/or scalp, left thereon for an appropriate period of time and then rinsed off with water. Thus, shampoos are a particularly preferred product form for the compositions.

Depending on the type of composition employed, one or more additional ingredients conventionally incorporated into hair treatment formulations may be included in the compositions Such additional ingredients include opacifiers such as polyethylene glycol distearate and ethylene glycol stearates, polymer latices, additional antimicrobial agents, foam boosters, perfumes, colouring agents, preservatives, viscosity modifiers, proteins, polymers, buffering or pH adjusting agents, moisturising agents, herb or other plant extracts and other natural ingredients.

### Shampoo Compositions

A particular preferred composition which may be used in the invention is a shampoo composition.

Such a shampoo composition will comprise one or more cleansing surfactants which are cosmetically acceptable and suitable for topical application to the hair. Further surfactants may be present as an additional ingredient if sufficient for cleansing purposes is not provided as emulsifier for any emulsified components in the composition, eg, emulsified silicones. It is preferred that shampoo compositions comprise at least one further surfactant (in addition to that used as emulsifying agent) to provide a cleansing benefit.

Suitable cleansing surfactants, which may be used singularly or in combination, are selected from anionic, amphoteric and zwitterionic surfactants, and the mixtures thereof. The cleansing surfactant may be the same surfactant as the emulsifier, or may be different.

Examples of anionic surfactants are the alkyl sulphates, alkyl ether sulphates, alkaryl sulphonates, alkanoyl isethionates, alkyl succinates, alkyl sulphosuccinates, N-alkyl sarcosinates, alkyl phosphates, alkyl ether phosphates, alkyl ether carboxylates, and alpha-olefin sulphonates, especially their sodium, magnesium, ammonium and mono-, di- and triethanolamine salts. The alkyl and acyl groups generally contain from 8 to 18 carbon atoms and may be unsaturated. The alkyl ether sulphates, alkyl ether phosphates and alkyl ether carboxylates may contain from 1 to 10 ethylene oxide or propylene oxide units per molecule.

Typical anionic surfactants for use in shampoos of the invention include sodium oleyl succinate, ammonium lauryl sulphosuccinate, ammonium lauryl sulphate, sodium dodecylbenzene sulphonate, triethanolamine dodecylbenzene sulphonate, sodium cocoyl isethionate, sodium lauryl isethionate and sodium N-lauryl sarcosinate. The most preferred anionic surfactants are sodium lauryl sulphate, triethanolamine monolauryl phosphate, sodium lauryl ether sulphate 1 EO, 2EO and 3EO, ammonium lauryl sulphate and ammonium lauryl ether sulphate 1EO, 2EO and 3EO.

Examples of amphoteric and zwitterionic surfactants include alkyl amine oxides, alkyl betaines, alkyl amidopropyl betaines, alkyl sulphobetaines (sultaines), alkyl glycinates, alkyl carboxyglycinates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in shampoos of the invention include lauryl amine oxide, cocodimethyl sulphopropyl betaine and preferably lauryl betaine, cocamidopropyl betaine and sodium cocamphopropionate.

The shampoo composition can also include co-surfactants, to help impart aesthetic, physical or cleansing properties to the composition. A preferred example is a nonionic surfactant, which can be included in an amount ranging from 0% to about 5% by weight of the total composition.

For example, representative nonionic surfactants that can be included in shampoo compositions include condensation products of aliphatic (C₈ - C₁₈) primary or secondary linear or branched chain alcohols or phenols with alkylene oxides, usually ethylene oxide and generally having from 6 to 30 ethylene oxide groups.

Other representative nonionics include mono- or di-alkyl alkanolamides. Examples include coco mono- or diethanolamide and coco mono-isopropanolamide.

Further nonionic surfactants which can be included in shampoo compositions are the alkyl polyglycosides (APGs). Typically, the APG is one which comprises an alkyl group connected (optionally via a bridging group) to a block of one or more glycosyl groups. Preferred APGs are defined by the following formula:

RO - (G) n

wherein R is a branched or straight chain alkyl group which may be saturated or unsaturated and G is a saccharide group.

R may represent a mean alkyl chain length of from about C₅ to about C₂₀. Preferably R represents a mean alkyl chain length of from about C₈ to about C₁₂. Most preferably the value of R lies between about 9.5 and about 10.5. G may be selected from C₅ or C₆ monosaccharide residues, and is preferably a glucoside. G may be selected from the group comprising glucose, xylose, lactose, fructose, mannose and derivatives thereof. Preferably G is glucose.

The degree of polymerisation, n, may have a value of from about 1 to about 10 or more. Preferably, the value of n lies in the range of from about 1.1 to about 2. Most preferably the value of n lies in the range of from about 1.3 to about 1.5.

Suitable alkyl polyglycosides for use in the invention are commercially available and include for example those materials identified as: Oramix NS10^{™} ex Seppic; Plantaren 1200^{™} and Plantaren 2000^{™} ex Henkel.

The total amount of surfactant (including any co-surfactant, and/or any emulsifier) in shampoo compositions is generally from 0.1 to 50% by weight, preferably from 5 to 30%, more preferably from 10% to 25% by weight of the total shampoo composition.

A cationic deposition polymer is a preferred ingredient in shampoo compositions, for enhancing conditioning performance of the shampoo. By deposition polymer is meant an agent which enhances deposition of the silicone component from the shampoo composition onto the intended site during use, ie, the hair and/or the scalp.

The deposition polymer may be a homopolymer or be formed from two or more types of monomers. The molecular weight of the polymer (in g/mol) will generally be between 5,000 and 10,000,000 typically at least 10,000 and preferably in the range 100,000 to about 2,000,000. The polymers will have cationic nitrogen containing groups such as quaternary ammonium or protonated amino groups, or a mixture thereof.

The cationic nitrogen-containing group will generally be present as a substituent on a fraction of the total monomer units of the deposition polymer. Thus, when the polymer is not a homopolymer it can contain spacer non-cationic monomer units. Such polymers are described in the CTFA Cosmetic Ingredient Directory, 3^{rd} edition. The ratio of the cationic to non-cationic monomer units is selected to give a polymer having a cationic charge density in the required range.

Suitable cationic deposition polymers include, for example, copolymers of vinyl monomers having cationic amine or quaternary ammonium functionalities with water soluble spacer monomers such as (meth)acrylamides, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general secondary and tertiary amines, especially tertiary, are preferred.

Amine substituted vinyl monomers and amines can be polymerized in the amine form and then converted to ammonium by quaternization.

The cationic deposition polymers can comprise mixtures of monomer units derived from amine- and/or quaternary ammonium-substituted monomer and/or compatible spacer monomers.

Suitable cationic deposition polymers include, for example:
- copolymers of 1-vinyl-2-pyrrolidine and 1-vinyl-3-methylimidazolium salt (eg chloride salt), referred to in the industry by the Cosmetic, Toiletry, and Fragrance Association, (CTFA) as Polyquaternium-16. This material is commercially available from BASF Wyandotte Corp. (Parsippany, NJ. USA) under the LUVIQUAT tradename (eg LUVIQUAT FX 370);
- copolymers of 1-vinyl-2-pyrrolidine and dimethylaminoethyl methacrylate, referred to in the industry (CTFA) as Polyquaternium-11. This material is available commercially from Gaf Corporation (Wayne, NJ, USA) under the GAFQUAT tradename (eg GAFQUAT 755N);
- cationic diallyl quaternary ammonium containing polymers including, for example, dimethyldiallylammonium chloride homopolymer and copolymers of acrylamide and dimethyldiallylammonium chloride, referred to in the industry (CTFA) as Polyquaternium 6 and Polyquaternium 7, respectively;
- mineral acid salts of amino-alkyl esters of homo-and copolymers of unsaturated carboxylic acids having from 3 to 5 carbon atoms, (as described in U.S. Patent 4,009,256);
- cationic polyacrylamides (as described in WO95/22311).

Other cationic deposition polymers that can be used include cationic polysaccharide polymers, such as cationic cellulose derivatives, cationic starch derivatives, and cationic guar gum derivatives.

Cationic polysaccharide polymers suitable for use in compositions include those of the formula:

A-0- [R-N⁺ (R¹) (R ²)(R³) X⁻],

wherein: A is an anhydroglucose residual group, such as a starch or cellulose anhydroglucose residual. R is an alkylene, oxyalkylene, polyoxyalkylene, or hydroxyalkylene group, or combination thereof. R¹, R² and R³ independently represent alkyl, aryl, alkylaryl, arylalkyl, alkoxyalkyl, or alkoxyaryl groups, each group containing up to about 18 carbon atoms. The total number of carbon atoms for each cationic moiety (i.e., the sum of carbon atoms in R¹, R² and R³) is preferably about 20 or less, and X is an anionic counterion.

Cationic cellulose is available from Amerchol Corp. (Edison, NJ, USA) in their Polymer JR (trade mark) and LR (trade mark) series of polymers, as salts of hydroxyethyl cellulose reacted with trimethyl ammonium substituted epoxide, referred to in the industry (CTFA) as Polyquaternium 10. Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from Amerchol Corp. (Edison, *NJ*, USA) under the tradename Polymer LM-200.

Other suitable cationic polysaccharide polymers include quaternary nitrogen-containing cellulose ethers (e.g. as described in U.S. Patent 3,962,418), and copolymers of etherified cellulose and starch (e.g. as described in U.S. Patent 3,958,581).

A particularly suitable type of cationic polysaccharide polymer that can be used is a cationic guar gum derivative, such as guar hydroxypropyltrimonium chloride (Commercially available from Rhodia (formerly RhonePoulenc) in their JAGUAR trademark series).

Examples are JAGUAR C13S, which has a low degree of substitution of the cationic groups and high viscosity. JAGUAR C15, having a moderate degree of substitution and a low viscosity, JAGUAR C17 (high degree of substitution, high viscosity), JAGUAR C16, which is a hydroxypropylated cationic guar derivative containing a low level of substituent groups as well as cationic quaternary ammonium groups, and JAGUAR 162 which is a high transparency, medium viscosity guar having a low degree of substitution.

Preferably the cationic deposition polymer is selected from cationic cellulose and cationic guar derivatives. Particularly preferred deposition polymers are JAGUAR C13S, JAGUAR C15, JAGUAR C17 and JAGUAR C16 and JAGUAR C162.

The cationic deposition polymer will generally be present at levels of from 0.001% to 5%, preferably from about 0.01% to 1%, more preferably from about 0.02% to about 0.5% by weight of the total composition.

### Active Agents

Active agents include, in addition to the insoluble metal pyrithione particles, other antifungal agents such as climbazole, piroctone olamine, selenium sulphide and ketoconazole.

Other suitable solid active agents include pigment particles, such as solid dyes or colorants suitable for application to hair, and metal colloids.

### Aesthetic Agents

Hair treatment compositions such as shampoos and conditioners are frequently opacified or pearlised to enhance consumer appeal.

Examples of opacifying agents include higher fatty alcohols (e.g. cetyl, stearyl, arachidyl and behenyl), solid esters (e.g. cetyl palmitate, glyceryl laurate, stearamide MEA-stearate), high molecular weight fatty amides and alkanolamides and various fatty acid derivatives such as propylene glycol and polyethylene glycol esters. Inorganic materials used to opacify hair treatment compositions include magnesium aluminum silicate, zinc oxide, and titanium dioxide.

Pearlescing agents typically form thin, platelet-type crystals in the composition, which act like tiny mirrors. This gives the pearl lustre effect. Some of the opacifying agents listed above may also crystallise as pearlescing agents, depending on the media in which they are used and the conditions employed.

Typical pearlescing agents may be selected from C16-C22 fatty acids (e.g. stearic acid, myristic acid, oleic acid and behenic acid), esters of C16-C22 fatty acid with alcohols and esters of C16-C22 fatty acid incorporating such elements as alkylene glycol units. Suitable alkylene glycol units may include ethylene glycol and propylene glycol. However, higher alkylene chain length glycols may be employed. Suitable higher alkylene chain length glycols include polyethylene glycol and polypropylene glycol.

Examples are polyethylene glycol mono or diesters of C16-C22 fatty acids having from 1 to 7 ethylene oxide units, and ethylene glycol esters of C16-C22 fatty acids. Preferred esters include polyethylene glycol distearates and ethylene glycol distearates. Examples of a polyethylene glycol distearate available commercially are EUPERLAN PK900^{™} (ex Henkel) or GENAPOL TS^{™} (ex Hoechst). An example of an ethylene glycol distearate is EUPERLAN PK3000^{™} (ex Henkel).

Other pearlescing agents include alkanolamides of fatty acids having from 16 to 22 carbon atoms, (e.g. stearic monoethanolamide, stearic diethanolamide, stearic monoisopropanolamide and stearic monoethanolamide stearate); long chain esters of long chain fatty acids (e.g. stearyl stearate, cetyl palmitate); glyceryl esters (e.g. glyceryl distearate), long chain esters of long chain alkanolamides (e.g. stearamide DEA distearate, stearamide MEA stearate), and alkyl (C18-C22) dimethyl amine oxides (e.g. stearyl dimethyl amine oxide).

Further suitable pearlescing agents include inorganic materials such as nacreous pigments based on the natural mineral mica. An example is titanium dioxide coated mica. Particles of this material may vary in size from 2 to 150 microns in diameter. In general, smaller particles give rise to a pearly appearance, whereas particles having a larger average diameter will result in a glittery composition.

Suitable titanium dioxide coated mica particles are those sold under the trade names TIMIRON (Merck) or FLAMENCO (Mearl).

The level of opacifying or pearlescing agent employed in compositions is generally from 0.01 to 20%, preferably 0.01 to 5%, more preferably from 0.02 to 2% by weight of the total composition.

Gas (e.g. air) bubbles represent another type of suspended phase that may be introduced into a hair treatment composition for aesthetic purposes. When evenly sized and homogeneously dispersed in the composition, these can enhance consumer appeal - a typical application is in a transparent or translucent composition such as a hair styling gel.

### Conditioners

Compositions for use in the invention may also be formulated as conditioners for the treatment of hair (typically after shampooing) and subsequent rinsing.

Such a conditioner will comprise one or more conditioning surfactants which are cosmetically acceptable and suitable for topical application to the hair.

Suitable conditioning surfactants are selected from cationic surfactants, used singly or in admixture. Examples include quaternary ammonium hydroxides or salts thereof, eg chlorides.

Suitable cationic surfactants for use in hair conditioners of the invention include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, cocotrimethylammonium chloride, and the corresponding hydroxides thereof. Further suitable cationic surfactants include those materials having the CTFA designations Quaternium-S, Quaternium-31 and Quaternium-18. Mixtures of any of the foregoing materials may also be suitable. A particularly useful cationic surfactant for use in hair conditioners of the invention is cetyltrimethylammonium chloride, available commercially, for example as GENAMIN CTAC^{™}, ex Hoechst Celanese.

In conditioners of the invention, the level of cationic surfactant is preferably from 0.01 to 10%, more preferably 0.05 to 5%, most preferably 0.1 to 2% by weight of the composition.

Conditioners of the invention advantageously incorporate a fatty alcohol. The combined use of fatty alcohols and cationic surfactants in conditioning compositions is believed to be especially advantageous, because this leads to the formation of a lamellar phase, in which the cationic surfactant is dispersed.

Representative fatty alcohols comprise from 8 to 22 carbon atoms, more preferably 16 to 20. Examples of suitable fatty alcohols include cetyl alcohol, stearyl alcohol and mixtures thereof. The use of these materials is also advantageous in that they contribute to the overall conditioning properties of compositions.

The level of fatty alcohol in conditioners of the invention is conveniently from 0.01 to 10%, preferably from 0.1 to 5% by weight of the composition. The weight ratio of cationic surfactant to fatty alcohol is suitably from 10:1 to 1:10, preferably from 4:1 to 1:8, optimally from 1:1 to 1:4.

### Conditioning Agents

The compositions which may be used in the invention may contain a conditioning agent. As used herein, the term conditioning agent includes any material which is used to give a particular conditioning benefit to hair and/or skin. For example, in compositions for use in washing hair, such as shampoos and conditioners, suitable materials are those which deliver one or more benefits relating to shine, softness, combability, wet-handling, anti-static properties, protection against damage, body, volume, stylability and manageability.

Preferred conditioning agents for use in the present invention include emulsified silicones, used to impart for example wet and dry conditioning benefits to hair such as softness, smooth feel and ease of combability.

Various methods of making emulsions of particles of silicones for use in the invention are available and are well known and documented in the art.

The viscosity of the silicone itself (not the emulsion or the final washing composition) preferably ranges from 10,000 cps to 5 million cps. The viscosity can be measured by means of a glass capillary viscometer as set out further in Dow Corning Corporate Test Method CTMOO4 July 20 1970.

Suitable silicones include polydiorganosiloxanes, in particular polydimethylsiloxanes which have the CTFA designation dimethicone. An example is dimethicone fluid having a viscosity of up to 100,000 centistokes at 25°C, which is available commercially from the General Electric Company as the Viscasil^{™} series and from Dow Corning as the DC 200^{™} series.

Aminofunctional silicones which have the CTFA designation amodimethicone, are also suitable for use in the compositions, as are polydimethyl siloxanes having hydroxyl end groups, which have the CTFA designation dimethiconol.

Also suitable are silicone gums. "Silicone gum" denotes polydiorganosiloxanes having a molecular weight of from 200,000 to 1,000,000 and specific examples include dimethicone gums, dimethiconol gums, polydimethyl siloxane/diphenyl/methylvinylsiloxane copolymers, polydimethylsiloxane/methylvinylsiloxane copolymers and mixtures thereof. Examples include those materials described in US Pat. No. 4,152,416 (Spitzer), and on General Electric Silicone Rubber product Data Sheet SE 30, SE 33, SE 54 and SE 76.

Also suitable for use in the present invention are silicone gums having a slight degree of cross-linking, as are described for example in WO 96/31188. These materials can impart body, volume and stylability to hair, as well as good wet and dry conditioning.

Preferred emulsified silicones for use in compositions have an average silicone particle size in the composition of less than 100, preferably less than 30, more preferably less than 20 microns, most preferably less than 10 microns.

Particle size may be measured by means of a laser light scattering technique, using a 2600D Particle Sizer from Malvern Instruments.

Suitable silicone emulsions for use in the invention are commercially available in a pre-emulsified form. This is particularly preferred since the pre-formed emulsion can be incorporated into the washing composition by simple mixing.

Examples of suitable pre-formed emulsions include emulsions DC2-1766 and DC2-1784, available from Dow Corning. These are emulsions of dimethiconol. Crosslinked silicone gums are also available in a pre-emulsified form, which is advantageous for ease of formulation. A preferred example is the material available from Dow Corning as DC X2-1787, which is an emulsion of cross-linked dimethiconol gum.

The amount of silicone incorporated into the compositions depends on the level of conditioning desired and the material used. A preferred amount is from 0.01 to about 10% by weight of the total composition although these limits are not absolute. The lower limit is determined by the minimum level to achieve conditioning and the upper limit by the maximum level to avoid making the hair and/or skin unacceptably greasy. We have found that an amount of silicone of from 0.5 to 1.5% by weight of the total composition, is a particularly suitable level.

A further preferred class of conditioning agents are peralk(en)yl hydrocarbon materials, used to enhance the body, volume and stylability of hair.

EP 567 326 and EP 498 119 describe suitable peralk(en)yl hydrocarbon materials for imparting stylability and enhanced body to hair. Preferred materials are polyisobutylene materials available from Presperse, Inc. under the PERMETHYL trade name.

The amount of per-alk(en)yl hydrocarbon material incorporated into the compositions depends on the level of body and volume enhancement desired and the specific material used. A preferred amount is from 0.01 to about 10% by weight of the total composition although these limits are not absolute. The lower limit is determined by the minimum level to achieve the body and volume enhancing effect and the upper limit by the maximum level to avoid making the hair unacceptably stiff. We have found that an amount of per-alk(en)yl hydrocarbon material of from 0.5 to 2% by weight of the total composition is a particularly suitable level.

### Optional Ingredients

Compositions useful in the invention may contain any other ingredients normally used in hair treatment formulations. These other ingredients may include viscosity modifiers, preservatives, colouring agents, polyols such as glycerine and polypropylene glycol, chelating agents such as EDTA, antioxidants, fragrances, and sunscreens. Each of these ingredients will be present in an amount effective to accomplish its purpose. Generally these optional ingredients are included individually at a level of up to about 5% by weight of the total composition.

Preferably, compositions useful in this invention also contain adjuvants suitable for hair care. Generally such ingredients are included individually at a level of up to 2%, preferably up to 1%, by weight of the total composition.

Among suitable hair care adjuvants, are:
(i) natural hair root nutrients, such as amino acids and sugars. Examples of suitable amino acids include arginine, cysteine, glutamine, glutamic acid, isoleucine, leucine, methionine, serine and valine, and/or precursors and derivatives thereof. The amino acids may be added singly, in mixtures, or in the form of peptides, e.g. di- and tripeptides. The amino acids may also be added in the form of a protein hydrolysate, such as a keratin or collagen hydrolysate. Suitable sugars are glucose, dextrose and fructose. These may be added singly or in the form of, e.g. fruit extracts. A particularly preferred combination of natural hair root nutrients for inclusion in compositions that may be used in the invention is isoleucine and glucose. A particularly preferred amino acid nutrient is arginine.
(ii) hair fibre benefit agents. Examples are:
   - ceramides, for moisturising the fibre and maintaining cuticle integrity. Ceramides are available by extraction from natural sources, or as synthetic ceramides and pseudoceramides. A preferred ceramide is Ceramide II, ex Quest. Mixtures of ceramides may also be suitable, such as Ceramides LS, ex Laboratoires Serobiologiques.

The invention will now be described with reference to the following non-limiting examples. In the examples and throughout this specification all percentages are by weight based on total composition unless indicated otherwise.

### Examples

Panellists consisting of males and females were used. Their scalps were assessed to confirm they were suffering from dandruff. The scalps were divided into two, from the centre of the forehead to the nape of the neck. Four sites of similar scalp condition, two on each side of the head, were identified.

Panellists were assessed for scalp condition then suitable subjects given a placebo shampoo (ie. non anti-dandruff) to use for approximately 5 weeks. They were then asked to return, having not washed their hair or applied products to it for 48 hours. They were re-assessed then tape stripped at one site on each side of the head (8 tapes per side). They then returned to be salon washed, by salon operators, 3 times per week with 1/2 of each head washed in the placebo and the other 1/2 in a shampoo containing 1% ZnPTO. After approximately 4 weeks, they were assessed and tape stripped again on adjacent sites to the first two.

Samples were collected by parting the hair and lightly swabbing with an alcohol swab to remove excess sebum. A total of eight sequential tapestrips were taken from each site. These were stored frozen until required for analysis. The sets of tapes from the panellists showing best improvement with ZnPTO were selected for lipid analysis

The corneocytes were removed from the tapes by sonicating in methanol and the free lipid was extracted from them in a chloroform/ methanol mixture, which was then dried under nitrogen. The residue was reconstituted in chloroform and the lipid species separated on amino-propyl bonded silica gel columns.

The lipids were then separated and quantified using HPTLC. The lipids analysed were total fatty acids, cholesterol, triglycerides and ceramides. The ceramides were separated into the individual ceramides that are normally seen. The covalently bound lipids were not analysed here. The protein content of the samples were measured and the lipid levels expressed as ng lipid per µg protein. The individual ceramides were also expressed as relative % of total ceramides.

The mean dandruff scores after treatment, for the 7 subjects, are decreased for both placebo and ZnPTO compared with before treatment, indicating improved scalp condition.

All the lipids measured are significantly increased after ZnPTO treatment, compared with the mass levels for before treatment. For the placebo a similar trend is seen, but the changes are only statistically significant for cholesterol, triglyceride and the total lipid levels. The two sites sampled before treatment are not statistically different from each other. The two sites sampled after treatment differed in that the side treated with ZnPTO had significantly higher levels of cholesterol and ceramide than the side treated with the placebo. The values are tabulated in Table 1.

**Table 1 Mass levels of lipid, in ng lipid per µg protein .**

| | Before Placebo | Before ZnPTO | After Placebo | After ZnPTO |
|---|---|---|---|---|
| Fatty Acids | 504.1 ± 319.8 | 364.9 ± 231.9 | 1341.8 ± 1254.3 | 1518.1± 748.6 |
| Cholesterol | 101.7 ± 3 1.0 | 108.9 ± 44.3 | 225.2 ± 93.0 | 478.3 ± 221.8 |
| Triglycerides | 202.03 ± 65.5 | 208.5 ± 77.1 | 974.9 ± 687.2 | 2539.7 ± 1911.2 |
| Ceramides | 315.3 ± 122.0 | 284.3 ± 62.4 | 458.5 ± 212.0 | 841.5 ± 243.9 |
| Total Lipid | 1123.4 ± 415.1 | 966.6 ± 282.5 | 3000.4 ± 1716.1 | 5377.6 ± 2056.0 |

The relative % fatty acid and cholesterol do not change with either treatment. Relative % ceramide significantly decreases after both placebo and ZnPTO treatment, accompanied by an increase in % triglyceride, which is significant for ZnPTO. The two sites sampled before treatment differ in that the sites before the placebo treatment have higher % fatty acids than the sites before ZnPTO treatment. There are no significant differences seen in the relative composition of the samples after treatment with placebo vs ZnPTO. The values are given in Table 2.

**Table 2 Relative % lipids in scalp stratum corneum.**

| | Before Placebo | Before ZnPTO | After Placebo | After ZnPTO |
|---|---|---|---|---|
| Fatty Acids | 41.3 ± 14.8 | 36.0 ± 12.5 | 39.2 ± 19.1 | 31.8 ± 16.6 |
| Cholesterol | 10.2 ± 4.5 | 11.6 ± 4.5 | 8.8 ± 4.3 | 1.4 |
| Triglycerides | 20.2 ± 9.6 | 21.4 ± 4.8 | 35.0 ± 16.0 | 43.0 ± 19.2 |
| Ceramides | 28.4 ± 6.1 | 31.0 ± 8.1 | 17.0 ± 4.1 | 16.4 ± 3.8 |

The changes in the relative % ceramides, seen between treatments are small. There is a statistically significant decrease in ceramide 6ii after both treatments accompanied by a decrease in ceramide 3 and an increase in ceramide 3a. These are significant only after the ZnPTO treatment. These values are represented in Table 3.

**Table 3 Ceramide as % of total ceramides**

| | Before Placebo | Before ZnPTO | After Placebo | After ZnPTO |
|---|---|---|---|---|
| Ceramide 1 | 12.6 ± 4.8 | 11.3 ± 4.1 | 14.3 ± 4.3 | 14.6 ± 5.3 |
| Ceramide 2 | 20.2 ± 5.0 | 19.4 ± 4.5 | 23.2 ± 5.2 | 22.3 ± 5.4 |
| Ceramide 3 | 11.5 ± 4.0 | 11.8 ± 3.9 | 7.9 ± 5.8 | 5.9 ± 4.0 |
| Ceramide 3a | 11.3 ± 1.4 | 12.4 ± 1.7 | 14.8 ± 4.2 | 19.2 ± 2.8 |
| Ceramide 4/5 | 21.9 ± 4.0 | 22.2 ± 3.0 | 19.2 ± 5.8 | 22.1 ± 5.9 |
| Ceramide 6i | 8.8 ± 1.7 | 9.2 ± 1.1 | 9.9 ± 7.8 | 8.0 ± 2.0 |
| Ceramide 6ii | 13.8 ± 1.7 | 13.8 ± 2.1 | 10.7± 2.7 | 8.0 ± 2.1 |

## Claims

1. Use of a metal pyrithione in a linse off composition for increasing the level of ceramides in the stratum corneum of the human scalp, wherein the overall level of ceramides increases and the total amount of ceramides 3 and 6ii decreases relative to the amount of ceramide 3a as a proportion of the total ceramide.

2. Use as claimed in Claim 1, wherein the metal pyrithione is zinc pyrithione.

3. Use as claimed in any one of Claims 1 to 3, wherein the composition is a shampoo composition.

4. Use as claimed in Claim 4, wherein the shampoo composition comprises from 0.001% to 5% by weight metal pyrithione.

## Patentansprüche

1. Verwendung eines Metallpyrithions in einer Ausspülzusammensetzung zum Erhöhen des Anteils an Ceramiden in dem Stratum corneum der menschlichen Kopfhaut, wobei sich der Gesamtanteil an Ceramiden erhöht und die Gesamtmenge an Ceramiden 3 und 6ii bezüglich der Menge an Ceramid 3a als ein Anteil des Gesamtceramids sinkt.

2. Verwendung nach Anspruch 1, wobei das Metallpyrithion Zinkpyrithion ist.

3. Verwendung nach einem der Ansprüche 1 bis 3, wobei die Zusammensetzung eine Schampoozusammensetzung ist.

4. Verwendung nach Anspruch 4, wobei die Schampoozusammensetzung 0,001 % bis 5 Gew.-% Metallpyrithion umfasst.

## Revendications

1. Utilisation d'une pyrithione métallique dans une composition rinçable pour augmenter le taux de céramides dans la couche cornée du cuir chevelu humain, dans laquelle le taux global de céramides augmente et la quantité totale de céramides 3 et 6ii diminue par rapport à la quantité de céramide 3a en tant que proportion de la teneur totale en céramides.

2. Utilisation selon la revendication 1, dans laquelle la pyrithione métallique est la pyrithione de zinc.

3. Utilisation selon l'une quelconque des revendications 1 à 3, dans laquelle la composition est une composition pour shampooing.

4. Utilisation selon la revendication 4, dans laquelle la composition pour shampooing comprend de 0,001 % à 5 % en poids de pyrithione métallique.
